# EUROPÄISCHE PATENTANMELDUNG

(11) **EP 0 536 646 A2**
(43) Veröffentlichungstag der Anmeldung: **14.04.1993**
(21) Anmeldenummer: 92116861.3
(22) Anmeldetag: 02.10.1992
(51) Int. Cl.: C12N 15/86, C12N 15/81

(54) **Neue recombinante Viren, deren Verwendung und deren Herstellung**

(30) Priorität: 07.10.1991 DE 4133038
(71) Anmelder: BOEHRINGER INGELHEIM INTERNATIONAL GmbH, D-55216 Ingelheim am Rhein (DE); GENENTECH, INC., South San Francisco California 94080 (US); IMPERIAL CANCER RESEARCH TECHNOLOGY LIMITED, London WC2A 3NL (GB)
(72) Erfinder: Nasmyth, Kim, Dr., A-1060 Wien (AT); Jones, Nicolas C., Dr., Hutton, Essex CM13 1LX (GB); Patel, G., Purley, Surrey CR2 1EN (GB)
(74) Vertreter: Laudien, Dieter, Dr.

(57) **Zusammenfassung**

Die vorliegende Erfindung betrifft in Viren und in Hefe replizierbare DNA-Sequenzen, enthaltend ein Virusgenom, eine Hefe-ARS-Sequenz, eine Hefe-CEN-Sequenz, mindestens ein Markergen, vorzugsweise jedoch ein oder zwei Markergene, und ein homogen austauschbares Insert, welches durch das zu exprimierende Gen ersetzt sein kann, ihre Transformanten in Hefe und in Viren sowie Verfahren zu ihrer Herstellung.

## Beschreibung

Große Viren, wie z.B. Baculoviren, haben breite Anwendung als eukaryotische Vektoren bei der Expression verschiedener Fremdproteine gefunden (siehe Übersichtsartikel 1 bis 3). In den meisten Fällen exprimieren Baculoviren-Vektoren in hohen Expressionsraten die gewünschten Proteine, die, mit nur einigen wenigen Ausnahmen, korrekt prozessiert werden und biologisch aktiv sind. Im Falle des Baculovirus basiert das Expressionssystem darauf, daß man das hochexprimierende Polyhedrin-Gen durch ein rekombinantes Gen ersetzt, das aus dem Polyhedrin-Promotor und der damit fusionierten, für das gewünschte Fremdprotein kodierenden Sequenz besteht. Da das Polyhedrin-Gen für die Replikation des Virus entbehrlich ist, ist das resultierende rekombinante Virus voll infektiös, und der aktivierte Polyhedrin-Promotor steuert in späten Phasen der Infektion die effiziente Transkription des Fremdgens.

Obwohl sich dieses System als sehr effizient und nützlich erwiesen hat, bleibt es wegen der Schwierigkeiten beim Identifizieren und Isolieren des rekombinanten Virus zeitraubend und mühsam.

Das für die Expression von Fremdgenen am breitesten verwendete Baculovirus ist das Autographa californica nuclear polyhedrosis virus (AcNPV), dessen Genom aus einem zirkulären doppelsträngigen DNA-Molekül von ca. 130 kb besteht (4). Auf Grund dieser Größe des Genoms ist es nicht möglich, Fremd-DNA mittels konventioneller Techniken, z.B. über Restriktionsendonuklease-Schnittstellen, zu inserieren. Die verwendete Methode beruht daher auf der homologen Rekombination zwischen Virus-DNA und einem geeigneten Transfervektor, die in Insektenzellen co-transfiziert werden (5 bis 9). Der Transfervektor besteht aus dem rekombinanten Gen, das stromabwärts vom Polyhedrin-Promotor eingefügt ist und von denselben Sequenzen flankiert wird, die das Polyhedrin-Gen im intakten Virus flankieren. In vivo Rekombination zwischen den homologen flankierenden Sequenzen in dem Virus und dem Transfervektor resultiert im Ersatz des Polyhedrin-Gens durch das Fremdgen. Die Schwierigkeit entsteht insbesondere aus der im allgemeinen niedrigen Rekombinationsfrequenz, die typischerweise nur zwischen 0,1 und 1 % liegt. Es ist daher erforderlich, das rekombinante Virus über einen sehr signifikanten Hintergrund vom Ursprungsvirus zu identifizieren.

Um die rekombinanten Viren zu identifizieren, wurden bisher folgende Methoden verwendet:
1.) Der mikroskopische Nachweis von Virusplaques, die aus Polyheder-negativ infizierten Zellen bestehen (10);
2.) Der Nachweis von rekombinanten Plaques durch Plaque-Hybridisierung an Sonden, die spezifisch für die Fremd-DNA sind, und anschließendes visuelles Screening (7 und 11);
3.) Das Screening von Plaques mittels Antikörpern (12).

Bei all diesen Methoden ist es erforderlich, mehrfache Plaque-Assays durchzuführen, bevor reines rekombinantes Virus erhalten wird.

Außerdem wurden kürzlich zwei Modifikationen beschrieben, die die Identifizierung der rekombinanten Plaques erleichtern:
In einem Fall enthält der Transfervektor zusätzlich zu dem durch den Polyhedrin-Promotor gesteuerten Fremdgen das bakterielle lacZ-Gen, das entweder von einem zweiten Viruspromotor oder von einem nichtviralen Promotor geleitet wird (13). Das rekombinante Virus, das durch homologe Rekombination entsteht, enthält auch dieses lacZ-Gen und kann daher identifiziert werden, indem Plaques auf β-Galaktosidase-Produktion untersucht werden.

Im zweiten Fall enthält das Ursprungsvirus eine nur einmal vorkommende Restriktionsendonuklease-Schnittstelle, die die Transfektion von linearer DNA eher zuläßt, als von zirkulärer viraler DNA (14). Dadurch wird die Rekombinationsfrequenz um das ca. 10-fache erhöht.

Es wurde nun gefunden, daß die vorstehend beschriebenen Nachteile der literaturbekannten Verfahren behoben werden können, wenn die für die Zellinfektion erforderliche infektiöse virale DNA durch homologe Rekombination einer in Viren und in Hefe replizierbaren bzw. propagierbaren DNA-Sequenz, enthaltend ein Virusgenom, eine Hefe-ARS-Sequenz, die für die autonome Replikation verantwortlich ist, eine CEN-Sequenz, die DNA-Molekülen mitotische Centromerfunktion verleiht und eine stabile niedrige Kopienzahl von Plasmid-DNA gewährleistet, mindestens ein Markergen, vorzugsweise jedoch ein oder zwei Markergene, und ein homogen austauschbares Insert, welches durch das zu exprimierende Gen ersetzt sein kann, erhalten wird.

Als Virusgenom kommt beispielsweise ein Baculovirus wie das Autographa californica nuclear polyhedrosis virus (AcNPV) (4),
als autonom replizierbare Hefe-DNA-Sequenz beispielsweise eine ARS-Sequenz wie die ARS1-Sequenz,
als zentromere Sequenz beispielsweise eine CEN-Sequenz wie die CEN4-Sequenz (43),
als selektionierbare Markergene beispielsweise das URA3-(44), can1-100-, ade2-1-, CY2-, TRP1-, LEU2-, HIS3- oder LYS2-Gen,
als homogen austauschbares Insert beispielsweise das Sup4-o-(42), CAN1-, URA3- oder LYS2-Gen,
als Eukaryoten Sf9-Zellen und
als zu exprimierendes Gen jedes Gen in Betracht, daß in Viren exprimiert werden kann, also beispielsweise die Interferongene, das CREB1- oder CREB2-Gen (41).

Gegenstand der vorliegenden Erfindung sind somit in Viren und in Hefe replizierbare DNA-Sequenzen, enthaltend ein Virusgenom, eine Hefe-ARS-Sequenz, eine Hefe-CEN-Sequenz, mindestens ein Markergen, vorzugsweise jedoch ein oder zwei Markergene, und ein homogen austauschbares Insert, welches durch das zu exprimierende Gen ersetzt sein kann, ihre Transformanten in Hefe und in Viren sowie Verfahren zu ihrer Herstellung.

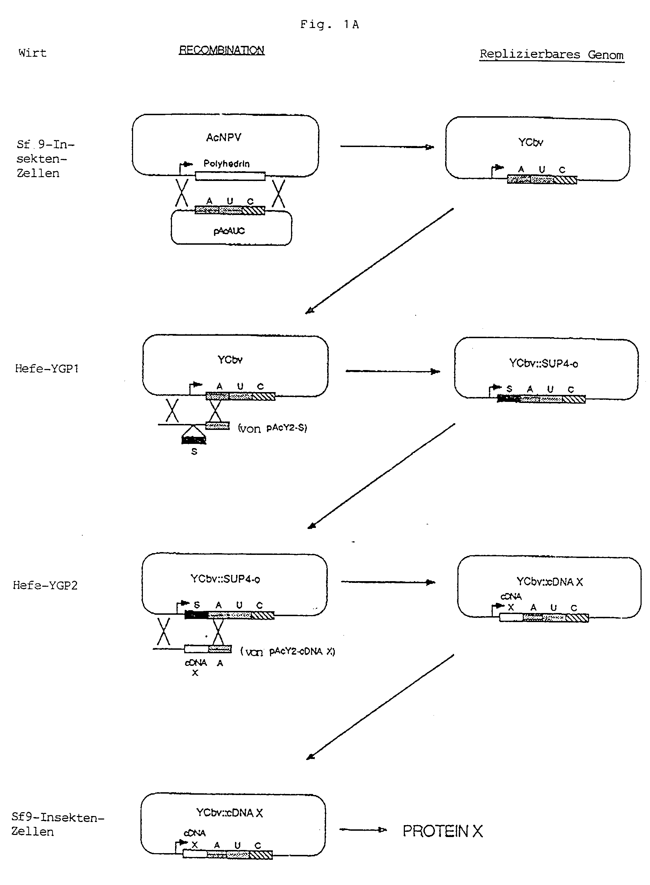

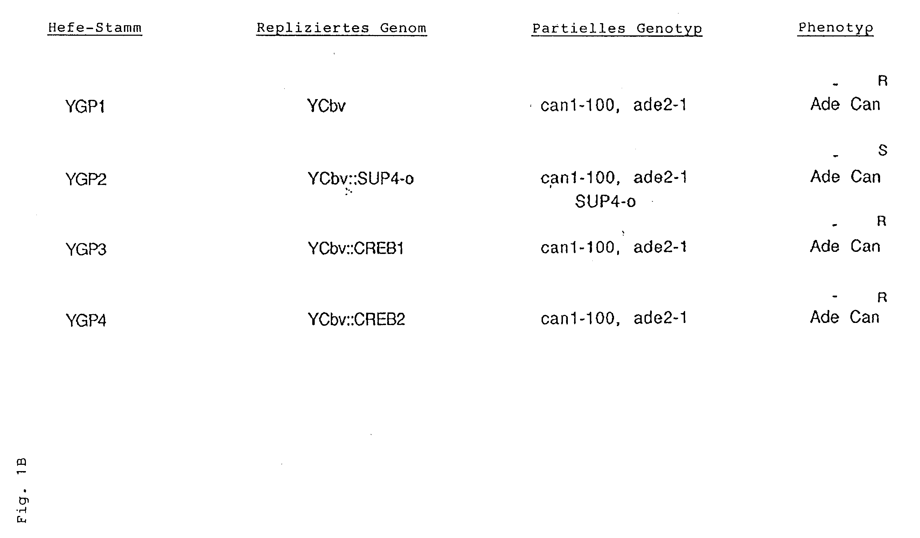

Am Beispiel der Expression zweier Mitglieder der Transkriptionsfaktor-Familie CREB/ATF wird das erfindungsgemäße Verfahren beispielsweise unter Verwendung des Baculovirus, einer ARS-Sequenz, die für die autonome Replikation von DNA in Hefe erforderlich ist (15 und 16), einer CEN-Sequenz, die DNA-Molekülen mitotische Centromerfunktion verleiht und eine stabile niedrige Kopienzahl von Plasmid-DNA gewährleistet (17), eines URA3-Markergens und gegebenenfalls eines selektionierbaren SuP4-o-Gens (18), durch homogenen Ersatz des vom Polyhedrin-Promotor gesteuerten Gens durch die entsprechenden -X-A-U-C-Sequenzen wie folgt durchgeführt (siehe Fig. 1A), wobei X für das zu exprimierende Gen, A für die TRP1/ARS1-Sequenzen, U für das URA3-Gen und C für das CEN4-Gen steht.

Im ersten Schritt wurde das Plasmid pBY3 durch Einfügung der TRP1/ARS1-, CEN4- und URA3-Sequenzen in das Bluescript-Plasmid (Stratagene) wie folgt hergestellt:
Ein 840 bp langes Fragment, das die TRP1/ARS1-Sequenzen (Fragment A) enthält, wurde zwischen die EcoRI- und HindIII-Schnittstellen des Bluescript-Plasmids, anschließend ein 1100 bp langes Fragment, das das URA3-Gen (Fragment U) enthält, in die HindIII-Schnittstelle und schließlich ein 1000 bp langes Fragment, das das CEN4-Gen (Fragment C) enthält, zwischen Xho1- und Kpn1-Schnittstellen eingefügt. Die so erhaltene Hefe-Kassette wurde als BamHI-Kpn1-Fragment entfernt und in die BamHI-Kpn1-Schnittstellen des Transfervektors pAcC4 (19) eingefügt, wobei das Plasmid pAcAUC erhalten wurde, das die Hefe-Kassette A-U-C enthält.

Der so erhaltene Vektor wurde zusammen mit Wildtyp-Baculovirus-DNA (AcNPV-DNA) in Sf9-Zellen co-transfiziert, wobei das rekombinante Baculovirus YCbv, welches visuell identifiziert und durch wiederholte Plaque-Assays gereinigt wurde, erhalten wurde (siehe auch (9)). Dieser Vektor enthält anstelle des Polyhedrin-Gens die vorstehend erwähnte Hefe-Kassette.

Im zweiten Schritt wurde aus den extrazellulären Viren des vorstehend beschriebenen Baculovirus (YCbv) virale DNA präpariert und mit dem Uracil-abhängigen S.cerevisiae Stamm y657 transformiert. Die transformierten Zellen werden durch Kultivieren in Abwesenheit von Uracil selektioniert. Hierbei wurde eine Anzahl von Ura⁺-Transformanten erhalten, von denen einige mittels Southern Blot-Analyse unter Verwendung von Gesamtvirus-DNA als Sonde auf das Vorhandensein von Baculovirus-Sequenzen untersucht wurden. Alle überprüften Ura⁺-Kolonien enthielten die erwarteten viralen Sequenzen. Von einem dieser Transformanten, welcher als YGP1 bezeichnet wurde und die Hefe-Kassette A-U-C enthält, wurde die Gesamt-DNA isoliert, mittels Saccharose-Gradientenzentrifugation teilweise gereinigt und in Sf9-Zellen transfiziert. 3 Tage später wurde der Kulturüberstand gewonnen und zur Infektion frischer Sf9-Zellen verwendet. Die mikroskopische Untersuchung der Zellen zeigte das Vorhandensein von infektiösem Virus an, was anschließend mittels Plaque-Assay bestätigt wurde. Aus den infizierten Zellen wurde Virus-DNA präpariert, von der sich herausstellte, daß sie ihre Fähigkeit, bei Transformation in Ura⁻-Hefe Uracil-Unabhängigkeit zu übertragen, beibehalten hat. Es ergab sich daher daraus klar, daß die rekombinante Baculovirus-DNA (YCbv) ohne erkennbare Schwierigkeit zwischen Hefe- und Insektenzellen hin und her pendeln kann.

Im dritten Schritt wurde, um den Nachweis von YCbv-Derivaten, die eine homologe Rekombination mit dem Transfervektor, der das zu exprimierende Fremdgen enthält, durchgemacht haben, zu erleichtern, ein zweites selektionierbares homogen austauschbares Insert in die YCbv-DNA, enthaltend in YGP1-Hefe, inseriert (siehe Fig. 1A). Dazu wurde das SUP4-o-Gen (Fragment S) ausgewählt, ein das ochre-Codon supprimierendes Allel eines tRNA^{Tyr}-Gens. In geeigneten Hefezellen, z.B. in y657, kann SUP4-o unter negative oder positive Selektion gesetzt werden.

Bei der erfindungsgemäßen Durchführung wurde ein Stamm verwendet, der ochre-Mutationen in den ADE2 und CAN1-Genen enthält. ADE2 kodiert für ein essentielles Enzym im Adenin-Biosyntheseweg (20 und 21). Mutationen in diesem Gen übertragen nicht nur die Adenin-Abhängigkeit des Wirts, sondern resultieren auch in der Akkumulation eines Zwischenprodukts des Adenin-Syntheseweges (Phosophoribosylaminoimidazol, AIR), der zu einer rosafarbenen Tönung der Zellkolonien führt. Das CAN1-Gen kodiert für das Enzym Argininpermease und macht die Zelle empfindlich gegenüber dem toxischen Arginin-Analogen Canavanin (22). Can⁻-Mutanten können keine funktionelle Argininpermease synthetisieren und sind daher resistent gegen Canavanin.

Um nun das SUP4-o-Gen in YCbv-DNA einzuführen, wurde ein Transfervektor, z. B. das Plasmid pAcY-S konstruiert, der das SUP4-o-Gen enthält, flankiert auf der einen Seite von Sequenzen, die stromaufwärts vom Polyhedrin-Translationsinitiationskodon liegen, und stromabwärts von den TRP1/ARS1-Sequenzen. Die folgende Transformation in YCbv enthaltende Hefe (YGP1) und die homologe Rekombination zwischen YCbv und dem Vektor führt zu der Insertion von SUP4-o in YCbv. Ein so erhaltenes Rekombinantes wurde als YGP2 bezeichnet. Dieses enthält die S-A-U-C-Kassette, wobei dessen Isolierung und Identifizierung weiter unten beschrieben wird.

Eine Übersicht über die verschiedenen Phänotypen von Zellen, abgeleitet von y657-Zellen, die das SUP4-o-Gen tragen oder nicht tragen, ist in Fig. 1B dargestellt. Die wesentlichen Merkmale sind hierbei, daß die Adenin-Unabhängigkeit eine positive Selektion auf Vorhandensein von SUP4-o gewährleistet, während die Canavanin-Resistenz eine starke Gegenselektion ermöglicht.

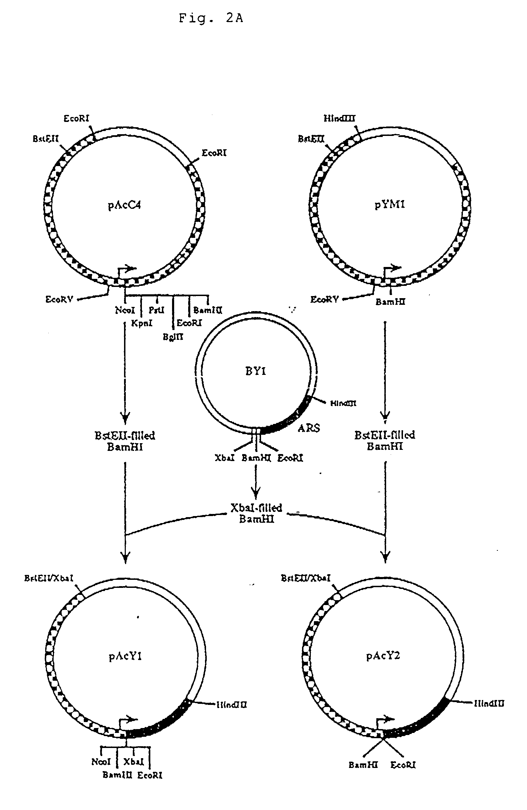

Die erforderlichen Vektoren wurden wie folgt konstruiert:
Den Transfervektor pAcY1 (siehe Fig. 2A) erhält man durch Einfügung der 840 bp langen TRP1/ARS1-Sequenz in die EcoRI-HindIII-Schnittstellen des Bluescript-Plasmids. Das so erhaltene Plasmid pBY1 wurde mit Xba1 verdaut, filled-in mit Klenow-Polymerase und erneut mit BamHI verdaut (Fragment 1). pAcC4-DNA (19) wurde verdaut mit BstEII, filled-in mit Klenow-Polymerase, erneut geschnitten mit BamHI und das 3kb-Fragment, das den Polyhedrin-Promotor und die 5'-flankierenden Sequenzen enthält, isoliert (Fragment 2). Das so erhaltene Fragment 2 wurde mit dem aus dem Plasmid pBY1 erhaltenen Fragment 1 ligiert, und das DNA-Fragment, das zwischen den NcoI-und EcoRI-Schnittstellen liegt, durch einen Polylinker ersetzt, wobei das Plasmid pAcY1 erhalten wurde.

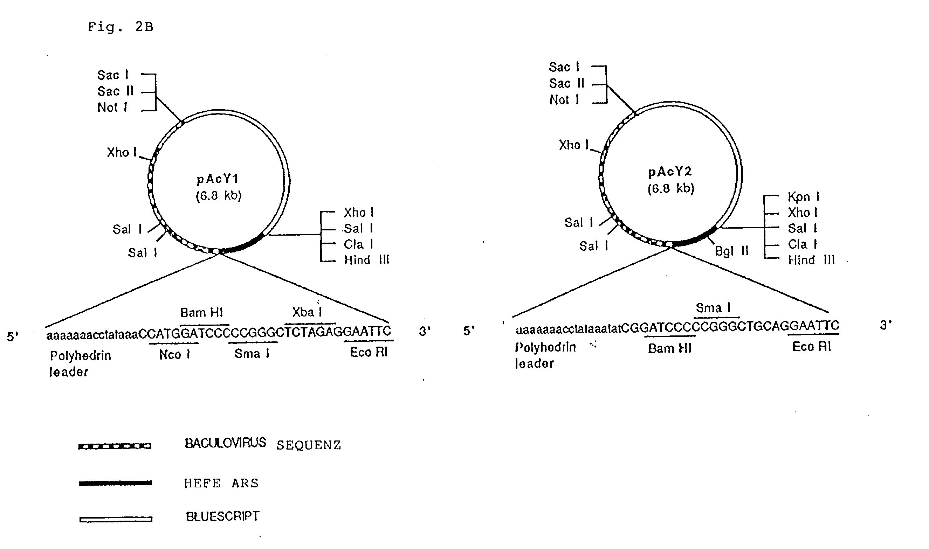

Die wesentlichen Merkmale dieses Vektors sind, daß er den Polyhedrinpromotor zusammen mit 3 kb der 5' flankierenden Baculovirus-Sequenz, einen Polylinker, der eine Anzahl von nur einmal vorkommenden Restriktionsstellen unmittelbar stromabwärts vom Promotor enthält, und Polyhedrin-5'-nichtkodierende Sequenzen, gefolgt von den Hefe-ARS-Sequenzen, die zur Konstruktion von YCbv verwendet wurden, enthält (siehe Fig. 2B). Die NcoI-Schnittstelle des Polylinkers enthält das Polyhedrin-ATG-Initiationskodon.

Der Transfer-Vektor pAcY2 wird analog erhalten, wobei jedoch das BstEII-BamHI-Fragment des Plasmids pAcYM1 (23) anstelle des obigen Fragments eingesetzt wurde.

Dieser Vektor ist bis auf den für die Insertion des Fremdgens verwendeten Polylinker (siehe Fig. 2A) mit dem Vektor pAcY1 sehr ähnlich. Das Polyhedrin-ATG-Kodon ist jedoch verlorengegangen und folglich muß das Initiationskodon durch die inserierte Fremdsequenz zur Verfügung gestellt werden (siehe Fig. 2B).

Daraus ergibt sich, daß in diesen beiden neuen Vektoren im Unterschied zu den vorbeschriebenen Vektoren, in denen das Insert auf beiden Seiten von Baculovirus-DNA flankiert ist, das Insert auf der 5'-Seite von viralen Sequenzen und auf der 3'-Seite von Hefe-ARS-Sequenzen flankiert ist.

Den Transfervektor pAcY2-S, welcher das SUP4-o-Gen enthält, erhält man durch Einfügung des 300 bp langen Fragmentes, das man durch Verdauen des Plasmids p464 mit EcoRI erhält und das das ochre-Suppressorgen SUP4-o enthält, in die EcoRI-Schnittstelle des Transfervektors pAcY2, welches sich kurz oberhalb der ARS1-Hefesequenz befindet.

Das so erhaltene pAcY2-S-Plasmid wurde zur Hefe-Transformation an der BglII-Schnittstelle, die sich in der ARS1-Sequenz befindet, und an der XhoI-Schnittstelle, die sich in der Baculovirus-Sequenz des Transfer-Vektors befindet, geschnitten und so mit dem bereits erhaltenen YGP1 rekombiniert.

Die so erhaltenen transformierten Zellen werden durch Kultivieren in Abwesenheit von Adenin selektiert und anschließend auf SD-Agar-Platten, denen Histidin, Tryptophan, Adenin und Leucin zugesetzt ist, per Kolonien gereinigt. Die einzelnen Kolonien werden hinsichtlich ihres Wachstums in Gegenwart von Canavanin getestet, dabei wurde die Kolonie zur weiteren Untersuchung ausgewählt, die gegenüber Canavanin sensitiv waren und als YGP2 bezeichnet.

Die ausgewählte YGP2-Kolonie weist das gewünschte Phenotyp in Gegenwart des tRNA Suppressors SUP4-o, nämlich die Adenin-Unabhängigkeit auf Grund der Unterdrückung der ochre-Mutation ade2-1 und die Canavanin-Sensität aufgrund der Suppression der CAN1-100-Mutation auf. Die Gegenwart des integrierten SUP4-o-Gens wurde ferner durch Southern-Hybridisierungsanalyse nachgewiesen.

Der so erhaltene Hefestamm YGP2 stellt einen gut geeigneten Rezipienten für die Insertion von Fremdsequenzen stromabwärts des Polyhedrin-Promotors mittels homologer Rekombination dar.

Im vierten Schritt wurde das neue System dazu verwendet, um Baculovirus-Rekombinanten zu isolieren, die zwei verschiedene Mitglieder der Transkriptionsfaktorfamilie CREB/ATF enthalten. Mitglieder dieser Familien binden spezifisch an die DNA-Sequenz 5' TT/GACGTCA 3', die über erhöhte cAMP-Konzentrationen (24) Transkriptionsaktivierung übertragen kann, und an das Adenovirus E1A-Protein (25). Es sind mindestens 10 verschiedene cDNA's beschrieben worden, die für Mitglieder dieser Familie kodieren (26 bis 30). Diese können alle als Homodimere an DNA binden, viele von ihnen können aber auch miteinander in Wechselwirkung treten und als Heterodimere binden. Die Aufklärung der hierdurch bedingten sehr signifikanten Komplexität könnte durch die Verfügbarkeit großer Mengen der einzelnen Mitglieder dieser Protein-Familie erleichtert werden. Für die durchgeführten Versuche wurden Rekombinanten ausgewählt, die CREB1- und CREB2- (CRE-BP1, ATF-2) cDNA's enthalten. Diese sind von speziellem Interesse, weil die CREB1-Aktivität von cAMP oder anderen Bedingungen, die die Kinase A-Aktivität erhöhen (31), moduliert und die CREB2-Aktivität durch das E1A-Protein moduliert wird (32 bis 34). In beiden Fällen wurden die cDNA's in den Vektor pAcY2 inseriert (siehe Fig. 2C) und anschließend zusammen mit einem Co-Transformations Plasmid, das das Hefe TRP1-Gen (45) enthält, in YGP2 Hefezellen eingeführt (siehe Fig. 1A):
Das pAcY2-CREB1-Plasmid erhält man durch Einfügung eines 1,6 kb langen EcoRI-Fragments, welches die CREB1-Sequenz einschließlich der 600 bp langen nicht-codierenden Sequenz am 3'-Ende enthält, in die EcoRI-Schnittstelle des Transfer-Vektors pAcY2 und
das pAcY2-CREB2-Plasmid erhält man durch Einfügung eines HincII-ClaI, filled-in Fragments, welches die CREB2-codierende Sequenz enthält, in die SmaI-Schnittstelle des Plasmids pAcY2.

Der erhaltene Transfer-Vektor pAcY2-CREB1 wurde mit Sall linearisiert, um die homologe Rekombination zu erleichtern, und zusammen mit einem Plasmid, das das Hefe TRP1-Gen enthält, mit YGP2-Sphäroplasten transformiert. Die erhaltenen Transformanten wurden durch Züchtung in Abwesenheit von Tryptophan selektioniert und die einzelnen Transformanten mittels Replica-Plattierung auf eine Canavanin enthaltende Platte auf Canavaninresistenz untersucht. Derartige resistente Transformanten entstehen von homologen Rekombinationen zwischen dem ortsansässigen YGP2- und pAcY2-CREB1-DNA's, wobei SUP4-o durch CREB1 ersetzt wird. Die Häufigkeit resistenter Transformanten wurde größenordnungsmäßig mit 80 % ermittelt. 12 verschiedene resistente Transformanten wurden mittels Southern-Hybridisierung auf den Ersatz des SUP4-o-Gens durch CREB1-Sequenzen untersucht, wobei einer dieser Transformanten als YGP3 (enthaltend YCbv::CREB1) bezeichnet wurde. Die Tatsache, daß dieses Ereignis in jedem einzelnen Transformanten stattgefunden hatte, zeigte die Effizienz des verwendeten Selektionssystems. Es sollte sich daher herausstellen, daß es nicht notwendig ist, routinemäßig die resistenten Kolonien durch Southern-Hybridisierung zu untersuchen.

Ein ähnliches Verfahren wurde zur Isolierung von CREB2 enthaltenden Rekombinanten benutzt, wobei jedoch der pAcY2-CREB2-Vektor mit XhoI linearisiert wurde. Einer der erhaltenen Transformanten wurde als YGP4 (enthaltend YCbv::CREB2) bezeichnet.

Die Gesamt-DNA von YGP3 und von YGP4 enthaltenden Hefezellen wurde unter Verwendung von Saccharose-Gradientenzentrifugation teilweise fraktioniert. Die Lage der Virus-DNA wurde mittels PCR-Analyse rasch bestimmt. Obwohl virale DNA über den gesamten Gradienten hinweg gefunden werden konnte, sedimentierte der Hauptanteil der DNA unter den verwendeten Bedingungen ungefähr in der Mitte des Gradienten (siehe auch Herstellung der Hefe-DNA).

Im Schritt 5 wurden die vorstehend erhaltenen DNA's mittels Lipofektion in Sf9-Zellen (siehe Fig. 1A) eingefügt, wobei die infizierten Zellen nach 3 Tagen deutlich sichtbar waren. Der Überstand wurde für darauffolgende Infektionen aufbewahrt und die Zellen auf das Vorhandensein von rekombinantem Protein untersucht.

Eine erfolgreiche Infektion konnte auch erreicht werden, wenn das nichtfraktionierte Gesamt-DNA-Präparat verwendet wurde. Es wurde jedoch gefunden, daß die Erfolgsrate unterschiedlich war und ein großer Bereich von DNA-Konzentrationen getestet werden mußte, um Aussicht auf Erfolg zu haben. Es wurde angenommen, daß die Unterschiede in der Erfolgsrate auf inhibierende Substanzen, die im Hefe-DNA-Präparat enthalten sind und die den Transfektionsprozeß beeinträchtigen, zurückzuführen sind. Im Gegensatz dazu wurden verläßliche Ergebnisse erhalten, wenn das Saccharosegradienten-Material verwendet wurde. Obwohl dieser Schritt nicht unbedingt notwendig ist, ist er bevorzugt.

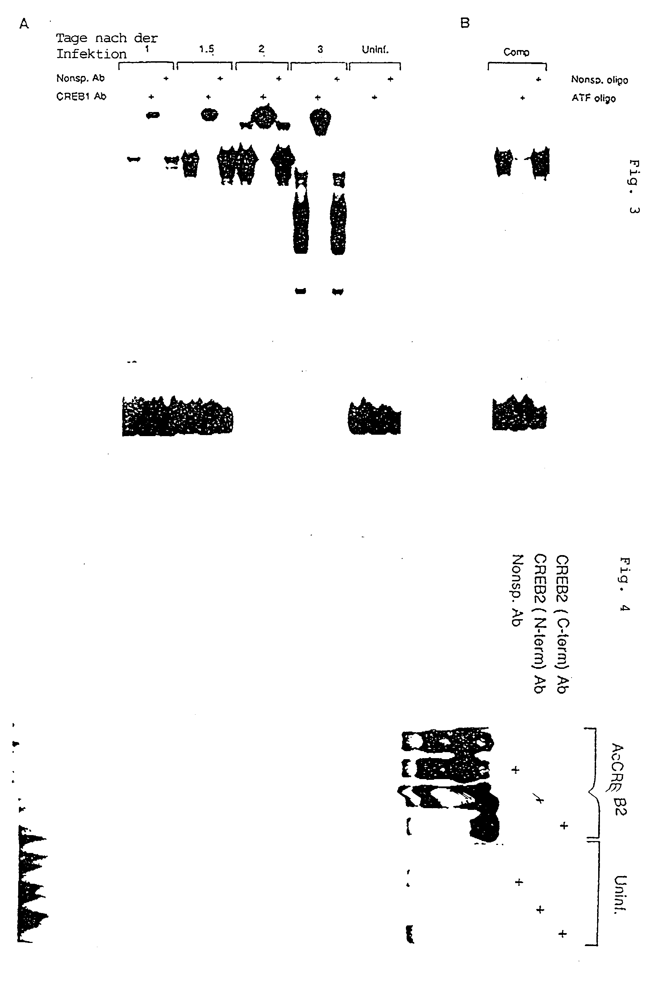

Die Expression des CREB1- und des CREB2-Proteins in virusinfizierten Sf9-Zellen wurde mittels elektrophoretischem Mobilitäts-Assay analysiert. Es wurden Gesamtzellextrakte hergestellt und auf spezifische DNA-Bindungsaktivität getestet, indem eine markierte Oligonukleotid-Sonde, enthaltend die Fibronectin-ATF/CRE-Bindungsstelle, verwendet wurde. Wie in Fig. 3A gezeigt wird, war am ersten Tag nach der Infektion der spezifische Protein-DNA-Komplex sichtbar; die Beweglichkeit des Komplexes wurde in Gegenwart eines Antikörpers gegen das C-terminale Peptid von CREB1 (35) verzögert, blieb hingegen durch einen unspezifischen Antikörper unbeeinträchtigt. Der Bindungsaktivitätsspiegel stieg während der nächsten beiden Tage signifikant. Es ergab sich jedoch, daß zu Zeitpunkten später als 2 Tage nach der Infektion viel vom CREB1-Protein abgebaut wurde und zu einer Reihe von schneller wandernden Komplexen führte. Bei längeren Inkubationszeiten konnte Bindungsaktivität auch in nichtinfizierten Extrakten festgestellt werden, wobei jedoch diese endogene Aktivität von dem CREB1-Antikörper nicht erkannt wurde. Die Spezifität der Bindung wurde durch Kompetition mit einem Überschuß von nichtmarkiertem Fibronectin-Oligonukleotid, das im Gegensatz zu unspezifischem Oligonukleotid gut um die Bindung konkurrierte, bestätigt werden (siehe Fig. 3B). Das Vorhandensein von CREB1-Protein in infizierten Extrakten und dessen Abbau später als 3 Tage nach der Infektion wurde mittels Western Blot bestätigt.

Extrakte von Zellen, die mit CREB2 enthaltenden Recombinanten infiziert sind, enthielten ebenfalls stark erhöhte Bindungsaktivitätsspiegel, welche spezifisch mit dem ATF-Oligonukleotid interagierten (siehe Fig. 4). 2 Tage nach der Infektion sieht man eine Anzahl von Komplexen, welche durch den infizierten Extrakt verursacht wurden. Die langsamste Komplex-Wanderung wird mit großer Sicherheit durch das intakte CREB2-Protein verursacht, zumal sich deren Beweglichkeit durch Antikörper verändert, die gegen Proteine gerichtet sind, die nahe zu dem N- oder C-Terminus von CREB2 lokalisiert sind. Zusätzlich zeigt die Western-Blot-Analyse die Anwesenheit des wandernden Proteins an der für CREB2 erwarteten Stelle. Die schneller wandernden Komplexe sind bedingt durch die proteolytische Spaltung des intakten Proteins. Diese werden nicht durch N-terminalen Antikörper erkannt, wohl aber durch den C-terminalen Antikörper. Wie beim CREB1 entstehen die kleineren Produkte erst viel später nach der Infektion.

Obwohl beide CREB Proteine in durch rekombinante Viren infizierten Zellen synthetisiert wurden, waren die akkumulierten Spiegel dieser Proteine signifikant voneinander verschieden. 2 Tage nach der Infizierung findet man 25 % CREB1-Protein bezogen auf das Gesamtprotein und nur annähernd 1-2 % CREB2-Protein. Diese Unterschiede sind vermutlich auf ihre unterschiedliche Stabilität zurückzuführen.

### Materialien und Methoden

### Zellen

Die Handhabung der Insektenzellinie Sf9 (ATCC Nr. CRL 1711) und die Produktion der "wild-type AcNPV" (Stamm E2) wurde bereits früher beschrieben (10).

Der benützte Stamm S. cerevisiae y657 (mat α, his3-11,15 trp1-1 ade2-1 leu2-3,112 ura3-52 can1-100 his4::HIS3) wurde uns von Andy Newman (38) zur Verfügung gestellt. Das Wachstum dieses Stammes oder seiner Derivate, welche Baculovirus/cen Plasmide enthalten, erfolgte in rich medium (Hefeextrakt/Pepton/Dextrose (YPD)) oder in einem synthetischen Minimum-Medium (SD), dem 20 µg/ml Histidin, 20 µg/ml Tryptophan, 20 µg/ml Adenin, 60 µg/ml Leucin und 10 mg/ml Casaminosäuren zugesetzt war. Die Transformaten, die gegen Canavanin resistent sind, wurden auf Platten, die 2 % Agar, SD, die üblichen Aminosäure-Zusätze und 60 µg/ml Canavaninsulfat (Sigma) enthalten, selektioniert. Das Hefewachstum erfolgte jeweils bei 30°C.

### Hefe Transformation

Die Hefe-Sphäroplasten wurden wie folgt hergestellt und transformiert (39):
Eine 50 ml Hefe-Kultur wurde in einem geeigneten Medium bis zu einer optischen Dichte von OD₆₀₀ = 0,5-1 gezüchtet. Die Zellen wurden 5 Minuten lang bei 2 K zentrifugiert, das erhaltene Pellet einmal mit destilliertem Wasser und einmal mit 1 M Sorbitol gewaschen und anschließend in 20 ml SCEM (1 M Sorbitol, 0,1 M Natriumzitrat pH 5,8, 10 mM EDTA und 30 mM β-Mercaptoethanol) und in 1000 Einheiten Lyticase (Sigma) resuspendiert. Die Zellsuspension wurde bei 30°C 20 bis 30 Minuten lang inkubiert, bis sich 90 % der Zellen zu Sphäroplasten umgewandelt hatten. Die Sphäroplasten wurden vorsichtig 6-7 Minuten lang bei 1 K zentrifugiert, das erhaltene Pellet mit 20 ml STC (1 M Sorbitol, 10 mM Tris/HCl pH 7,5 und 10 mM Calciumchlorid) gewaschen und schließlich in 2 ml STC suspendiert. 5 µg des Transvektors, der die relevante Fremd-DNA enthält, wurde innerhalb der Polyhedrin-Sequenzen und entweder innerhalb der ARS-Sequenz oder unmittelbar unterhalb der ARS-Sequenz geschnitten. Das geschnittene Plasmid wurde mit Ethanol ausgefällt und zur Transformation mit 100 µl der erhaltenen Sphäroplasten verwendet. Die Hefe-Plasmid-DNA-Mischung wurde 10 Minuten lang bei Raumtemperatur stehen gelassen, anschließend wurde 1 ml PEG-Lösung (10 mM Tris/HCl pH 7,5, 10 mM Calciumchlorid und 20 % PEG 8000) hinzugefügt und weitere 10 Minuten lang inkubiert. Die Sphäroplasten wurden 6-7 Minuten lang bei 1 K zentrifugiert, das erhaltene Pellet in 150 µl SDS-Puffer (1 M Sorbitol, 6,5 mM Calciumchlorid, 0,25 % Hefeextrakt (Difco) und 0,5 % Bactopepton (Difco)) suspendiert und 15-30 Minuten lang bei 30°C inkubiert. Anschließend wurden 8 ml geschmolzener Topagar, der die relevanten Zusätze enthielt, zu der Sphäroplasten-Suspension gegeben und diese unmittelbar auf der Oberfläche einer Agar-Platte, die SD-Medium mit den Zusätzen Histidin, Leucin, Adenin und Tryphophan enthielt, verteilt. Zur Cotransformation wurde 0,5 µg des das TRP1-Gen enthaltenden Plasmids zu den geschnittenen Transfer-Vektoren gegeben. Die Selektion wurde in Tryptophan freiem Medium durchgeführt.

### Herstellung der Hefe-DNA

Die Gesamt-Hefe-DNA wurde wie in (40) beschrieben präpariert:
250 ml YGP3 oder YGP4 Hefe-Zellen werden in SD-Medium, das geeignete Zusätze und Casaminosäuren enthält, bis zu einer optischen Dichte von OD₆₀₀ = 1-2 gezüchtet. Die Sphäroplasten wurden wie bei dem Transformationsverfahren beschrieben präpariert und sachte in 15 ml Lyse-Puffer enthaltend 4,5 M GuHCl, 0,1 M EDTA pH8, 0,15 M NaCL und 0,05 % Sarkosyl resuspendiert. Nach 10 Minuten bei 65°C wurde die Suspension auf Raumtemperatur gekühlt, die DNA mit 15 ml kaltem Ethanol ausgefällt und durch 10-minütige Zentrifugation bei 10.000 rpm in einem Sorvall SS-34-Rotor abgetrennt. Das erhaltene Pellet wurde in 10 ml 0,1 M Tris pH 7.5, 0,01 M EDTA und 50 µl RNAse A (10 mg pro ml) resuspendiert. Nach 60-minütiger Inkubation bei 37°C wurde 150 µl Proteinase K (10 mg/ml) zugegeben und weitere 30 Minuten bei 65°C inkubiert. Die DNA wurde 2 x mit Phenol extrahiert, mit Ethanol ausgefällt und in TE (10 mM Tris und 1 mM EDTA) suspendiert.

Die Gesamt-DNA, die bei den meisten der beschriebenen Experimente verwendet wurde, wurde durch Saccharose-Gradienten-Zentrifugation fraktioniert. Hierzu wurde die DNA oben auf einen 5-20%igen Saccharose-Gradienten in 200 mM NaCl, 10 mM Tris pH 7,5 und 2 mM EDTA eingebracht. Die Gradienten wurden 3 Stunden lang bei 35K und 20°C in einem SW40-Rotor zentrifugiert. Unter diesen Umständen wanderte der größte Teile der Baculovirus-DNA abwärts bis zur Hälfte des Gradienten. Der Gradient wurde fraktioniert und 2 Volumen kaltes Ethanol zu den Fraktionen, die die Baculovirus-DNA enthalten, zugefügt. Die ausgefällte DNA wurde erneut in TE (10 mM Tris, 1 mM EDTA) suspendiert und so zur Transfektion von Insektenzellen verwendet.

### Transfektion von Insektenzellen

Saccharose-Gradienten fraktionierte DNA wurde in Sf9-Zellen unter Verwendung von Lipofectin (Gibco) eingeschleust. Wasser wurde zu 5-10 µl fraktionierter DNA bis zu einem Volumen von 12 µl gegeben. Das gleiche Volumen Lipofectin-Puffer, enthaltend 8 µl Lipofectin, wird mit 4 µl Wasser und zu der DNA gegeben. Die beiden Lösungen wurden vorsichtigt miteinander vermischt und nach 15-minütiger Inkubation bei Zimmertemperatur auf eine 35 mm Zellkulturplatte, die 1.5 x 10⁶ Sf6-Zellen in 1 ml serumfreiem Medium enthält, gegeben. Nach 16 Stunden bei 28°C wurde das Medium entfernt und durch frisches 10 % FCS enthaltendes Medium ersetzt. Am 3. oder 4. Tag sind die Zellen sichtbar infiziert, zur gleichen Zeit wurden die Zellen zu analytischen Zwecken geerntet und der Überstand zur erneuten Infektion von frischen Zellen gewonnen.

### Elektrophoretischer Mobilitäts-Essay

Infizierte oder "lipoinfizierte" Sf9-Zellen von einer 35 mm Platte wurden mit PBS gewaschen und die Zellen erneut in 100 µl Extraktions-Puffer, welcher 10 mM Hepes pH 7,9, 1,5 mM MgCl₂, 0,1 mM EGTA, 5 % Glycerin, 0,5 mM DTT und 0,5 mM PMSF enthält, gewaschen. Nach 5 Minuten bei 0°C wurde das Lysat 10 Sekunden lang zentrifugiert und der Überstand in ein frisches Röhrchen gegeben. Das erhaltene Pellet wurde erneut in 50 µl Extraktionspuffer, welcher 0,35 M NaCl enthält, suspendiert und 15 Minuten lang bei 4°C inkubiert. Nachdem Zelltrümmer durch 10-minütiges Zentrifugieren entfernt worden war, wurden 45 µl des Überstandes mit 100 µl des "low salt" Lysats versetzt, wobei der gewünschte Zellextrakt mit einer Endsalzkonzentration von 0,1 M erhalten wurde. Elektrophoretische Mobilitäts-Assays unter Verwendung eines Oligonucleotids, welches die Fibronectin ATF/CREB-Bindungsstelle enthält, wurde bereits früher beschrieben (41). 1 µl Zellextrakt wurde in jedem Assay benützt. Der Antikörper wurde 5 Minuten nach Einleiten der Bindungsreaktion hinzugegeben um die Wirksamkeit der spezifischen Antikörper auf die Mobilität der Komplexe zu testen. Hierbei wurde folgender Antikörper benutzt:
Der CREB1-Antikörper ist literaturbekannt (35) und erkennt die 10 terminalen Aminosäuren des menschlichen CREB1-Proteins. Der N-terminale CREB2-Antikörper (NJ2) erkennt ein Peptid, welches die Aminosäuren 85-96 des menschlichen CREB2-Proteins enthält, und der C-terminale Antikörper (NJ4) ein Peptid, das die Aminosäuren 490-505 enthält.

### Legende zu den Figuren

Figur 1A enthält eine schematische Darstellung der Strategie zur Isolierung des recombinanten Baculovirus unter Verwendung von S.cerevisiae als Zwischenwirt (siehe auch Beschreibung ab Seite 5). Die ARS-, URA- und CEN-Hefeelemente wurden als A, U bzw. C bezeichnet.

Figur 1B stellt den Phenotyp der y657-Hefederivate dar, die das Sup4-o-Gen enthalten oder nicht enthalten, wobei der y657-Stamm als Ausgangsmaterial dient.
YGP2 enthält episomisch aufrechterhaltene Baculovirus-DNA, in die stromabwärts vom Polyhedrin-Promotor die Hefe-Sequenzen Sup4-o, ARS, URA und CEN eingefügt sind (siehe Fig. 1A).

YGP3 und YGP4 sind YGP2-Derivate, in denen die replizierbaren Genome die CREB1- und CREB2-cDNA-Sequenzen an Stelle des Sup4-o-Gens enthalten.

Figur 2A gibt die Konstruktionsmerkmale der Transfervektoren pAcY1 und pAcY2 wieder. Die durchgezogenen, schraffierten und leeren Plasmidregionen stellen die Hefe-ARS-, Baculovirus- bzw. Basisplasmidsequenzen dar.

Fig. 2B stellt die Restriction-Endonuclease-Karte der Transfervektoren pAcY1 und pAcY2 dar. Die Plasmide enthalten die Baculovirus Polyhedrin-Promotor- und Leadersequenz, die oberhalb durch virale Sequenzen und unterhalb durch Hefe TRR/ARS-Sequenzen flankiert sind. Die flußabwärts zu der Leadersequenz liegenden Schnittstellen, die zur Einfügung von fremden codierenden Sequenzen geeignet sind, sind gekennzeichnet.

Im Falle von pAcY1 enthält die Endversion dieses Transfervektors ein Oligonukleotid, das zwischen den NcoI- und EcoRI-Schnittstellen liegt und die angegebene Sequenz aufweist.

Figur 2C stellt die Restriction-Endonuclease-Karte der pAcY1-Derivate dar, die Inserte der menschlichen CREB1- und CREB2-(CRE-BP1)-cDNA-Sequenzen enthalten.

Figur 3 gibt die Gel-Retardation-Analyse des durch Baculovirus synthetisierte CREB1 wieder, das an ein Oligonucleotid gebunden ist, das die Fibronectin-CRE/ATF-Bindungsstelle enthält. Sf9-Zellen wurden infiziert mit einer Baculovirus-CREB1-Recombinante und jeweils der gesamte Extrakt 1, 1,5, 2 und 3 Tage nach der Infektion präpariert. Aliquote jedes Extraktes wurden mit [³²P]-markierten CRE enthaltendem Oligonukleotid in Abwesenheit oder Gegenwart von gegen CREB1 spezifischen oder nichtspezifischen Antikörper (A) oder einem 100-fachen molaren Überschuß an Fibronectin-Oligonukleotid oder einem nichtspezifischen Oligonukleotid (B) getestet.

Figur 4 gibt die Gel-Retardations-Analyse des durch Baculovirus synthetisierten CREB2 wieder, das an CRE/ATF enthaltendes Oligonukleotid gebunden ist. Sf9-Zellen wurden infiziert mit Baculovirus-CREB2-Rekombinanten und der gesamte Extrakt wurde 2 Tage später präpariert. Der Extrakt wurde auf seine Bindung gegen [³²P]-markiertes Fibronectin-Oligonukleotid in Abwesenheit oder Gegenwart von zwei verschiedenen CREB2 spezifischen Peptidantikörpern oder einem nichtspezifischen Antikörper hin untersucht. Eine ähnliche Analyse wurde mit einem Extrakt nichtinfizierter Zellen durchgeführt.

### Liste der Referenzen:

(1) Luckow, V.A. und Summers, M.D., Virology 167, 56-71 (1988)
(2) Miller, L.K., Ann. Rev. Microbiol. 42, 177-199 (1988)
(3) Maeda, S., Ann. Rev. Entomology 24, 351-372 (1988)
(4) Burgess, S., J. Gen. Virol. 37, 501-510 (1977)
(5) Pennock, G.D., Shoemaker, C., Miller, L.K., Mol. Cell. Biol. 4 399-406 (1984)
(6) Smith, G.E., Fraser, M.J. and Summers, M.D., J. Virol. 46, 584-593 (1983)
(7) Greenfield, C., Patel, G., Clark, S., Jones, N.C., Waterfield, M.D., EMBO J. 7, 139-146 (1988)
(8) Patel, G. und Stabel, S., Cell Signalling 1, 227-250 (1989)
(9) Patel, G. und Jones, N.C., Nucl. Acids. Res. 18, 2909-2915 (1990)
(10) Summers, M.D. und Smith, G.E., Texas Agricultural Experiment Station Bulletin No. 1555 (1987)
(11) Pen, J., Welling, G.W. und Welling-Webster, S., Nucl. Acids Res. 17, 451 (1989)
(12) Capone, J., Gene Anal. Tech. 6, 62-66 (1989)
(13) Zuidema, D., Schouten, A., Usmany, M., Maule, A.JH., Belsham, G.J., Roosien, J., Klinge-Roode, E.C., van Lent, J.W.M. und Vlak, J.M., J. Virol. 71, 2201-2209 (1990)
(14) Kitts, P.A., Ayres, M.D. und Possee, R.D., Nucl. Acids. Res. 18, 5667-5672 (1990)
(15) Brewer, B.J. und Fangman, W.L., Cell 51, 463-471 (1987)
(16) Stinchcomb, D.T., Struhl, K. und Davis, R.W., Nature 282, 39-43 (1979)
(17) Fitzgerald-Hayes, M., Yeast 3, 187-200 (1987)
(18) Gesteland, R.F., Wolfner, M., Grisafi, P., Fink, G., Botstein, D. und Roth, J.R., Cell 7, 381-390 (1976)
(19) Luckow, V.A. und Summers, M.D., Bio Technology 6, 47-55 (1988)
(20) Silver, J.M. und Eaton, N.R., Biochem. Biophys. Res. Comm. 34, 301-305 (1969)
(21) Woods, R.A., Mol. Gen. Genet. 105, 314-316 (1969)
(22) Hoffmann, W., J. Biol. Chem. 260, 11831-11837 (1985)
(23) Matsuura, Y., Possee, R.D., Overton, H.A. und Bishop, D.H.L., J. Gen. Virol. 68, 1233-1250 (1987)
(24) Montminy, M.R., Savarino, K.A., Wagner, J.A., Mandel, G. und Goodman, R.H., Proc. Natl. Acad. Sci. USA. 86, 6682-6686 (1986)
(25) Lee, K.A.W., Hai, T.Y., Shivaraman, L., Thimmappaya, B., Hurst, H.C., Jones, N.C. und Green, M.R., Proc. Natl. Acad. Sci. USA. 84, 8355-8359 (1987)
(26) Hoeffler, J.P., Meyer, T.E., Yun, Y., Jameson, J.L. und Habener, J.F., Science 242, 1430-1433 (1988)
(27) Gonzalez, G.A., Yamamoto, K.K., Fischer, W.H., Karr, K., Manzel, P., Biggs III, W., Vale, W.W. und Montminy, M.R., Nature 337, 749-752 (1989)
(28) Hai, T.Y., Liu, F., Coukos, W.J. und Green, M.R., Genes Devel. 3, 2083-2090 (1989)
(29) Maekawa, T., Sakura, H., Kanei-Ishii, C., Sudo, T., Yoshimura, T., Fujisawa, J., Yoshida, M. und Ishii, S., EMBO J. 8, 2023-2028 (1989)
(30) Yoshimura, T., Fujisawa, J. und Yoshida, M., EMBO J. 9, 2537-2542 (1990)
(31) Gonzalez, G.A. and Montminy, M.R., Cell 59, 675-680 (1989)
(32) Liu, F. und Green, M.R., Cell 61, 1217-1224 (1990)
(33) Maekawa, T., Matsuda, S., Fujisawa, J., Yoshida, M. and Ishii, S., Oncogene 6, 627-632 (1991)
(34) Flint, K. und Jones, N.C., Oncogene, im Druck
(35) Hurst, H.C., Masson, N., Jones, N.C. und Lee, K.A.W., Mol. Cell. Biol. 10, 6192-6203 (1990)
(36) Ward, M., Scott, R.J., Davey, M.R., Clothier, R.H., Cocking, E.C. und Balls, M., Somat. Cell. Mol. Genet. 12, 101-109 (1986)
(37) Gnirke, A., Barnes, T.S., Patterson, D., Schild, D., Featherstone, T. and Olsen, M.V., Embo J. 10, 1629-1634 (1981)
(38) Newman, A. und Norman, C., Cell 65, 115-123 (1991)
(39) Burgess, P.M.J. und Percival, K.J., Anal. Biochem. 163, 391-397 (1987)
(40) Holm, C., Meeks-Wagner, D.W., Fangman, W.L. und Botsteon, D., Gene 42, 169-173 (1986)
(41) Benbrook, D.M. und Jones, N.C., Oncogene 5, 295-302 (1990)
(42) Rotstein, R., Cell. 17, 185-190 (1979)
(43) Mann, C. und Davis, R. W., Mol Cell. Biol. 6, 241-245 (1986)
(44) Rose, M., Grisafi, P. und Botstein, D., Gene 29, 113-124 (1984)
(45) Tschumber, G. und Carbon, J., Gene 10, 157-166 (1980)

## Patentansprüche

1. In Hefe und in Viren replizierbare DNA-Sequenzen, dadurch gekennzeichnet, daß diese ein Virusgenom, eine Hefe-ARS-Sequenz, eine Hefe-CEN-Sequenz, mindestens ein Markergen und ein homogen austauschbares Insert, welches durch das zu exprimierende Gen ersetzt sein kann, enthalten.

2. DNA-Sequenzen gemäß Anspruch 1, dadurch gekennzeichnet, daß als Virusgenom das eines großen Virus wie des Baculovirus verwendet wird.

3. DNA-Sequenzen gemäß Anspruch 1, dadurch gekennzeichnet, daß als Markersequenz die URA3-, can1-100-, ade2-1-, CY2-, TRP1-, LEU2-, HIS3- oder LYS2-Sequenz verwendet wird.

4. DNA-Sequenzen gemäß Anspruch 1, dadurch gekennzeichnet, daß als homogen austauschbares Insert das Sup4-o-, CAN1-, URA3- oder LYS2-Gen verwendet wird.

5. DNA-Sequenzen gemäß Anspruch 1 und 2, dadurch gekennzeichnet, daß die Baculovirus-DNA anstelle des Polyhedrin-Gens die Fragmente
- A - U - C -,
- S - A - U - C - und
- X - A - U - C - enthält, wobei
A für die TRP1/ARS1-Sequenzen,
U für das URA3/Gen,
C für das CEN4-Gen,
S für das Sup4-o-Gen und
X für das zu exprimierende Gen steht.

6. DNA-Fragmente der Formeln
- A - U - C -,
- S - A - U - C - und
- X - A - U - C - ,
in denen
A, U, C, S und X wie im Anspruch 5 definiert sind.

7. DNA-Sequenzen gemäß den Ansprüchen 5 und 6, dadurch gekennzeichnet, daß X die für ein Interferon, CREB1 oder CREB2 codierende Sequenz darstellt.

8. Transformanten, enthaltend eine DNA-Sequenz gemäß den Ansprüchen 1 bis 7.

9. Verfahren zur Herstellung der DNA-Sequenzen gemäß den Ansprüchen 1 bis 5, dadurch gekennzeichnet, daß in Hefe ein Virusgenom mit einer Hefekassette, enhaltend eine Hefe-ARS-Sequenz, eine Hefe-CEN-Sequenz, mindestens eine Markersequenz und ein homogen austauschbares-Insert repliziert wird und gewünschtenfalls anschließend ein homogen austauschbares Insert durch ein zu exprimierendes Gen mittels homogener Rekombination ersetzt wird.

10. Verfahren zur Herstellung der DNA-Fragmente gemäß Anspruch 6, dadurch gekennzeichnet, daß die einzelnen Fragmente miteinander ligiert und anschließend in einem geeigneten Wirt propargiert werden.

11. Verfahren zur Herstellung der Virus-Transformanten gemäß Anspruch 8, dadurch gekennzeichnet, daß ein Virus mit den DNA-Sequenzen gemäß den Ansprüchen 1 bis 7 rekombiniert wird.

12. Verfahren zur Herstellung der Hefe-Transformanten gemäß Anspruch 8, dadurch gekennzeichnet, daß Hefe mit den DNA-Sequenzen gemäß den Ansprüchen 1 bis 7 transformiert wird.
